# EUROPÄISCHE PATENTSCHRIFT

(11) **EP 1 342 488 B1**
(45) Veröffentlichungstag und Bekanntmachung des Hinweises auf die Patenterteilung: **26.04.2017**
(21) Anmeldenummer: 03004035.6
(22) Anmeldetag: 24.02.2003
(51) Int. Cl.: A61N 5/10

(54) **Verfahren und Vorrichtung zur wiederholt gleichen Relativpositionierung eines Patienten**
Method and device for repeated relative repositioning of a patient
Procédé et dispositif pour le repositionnement relatif répété d'un patient

(30) Priorität: 07.03.2002 DE 10210050
(43) Veröffentlichungstag der Anmeldung: 10.09.2003
(73) Patentinhaber: Siemens Healthcare GmbH, 91052 Erlangen (DE)
(72) Erfinder: Kagermeier, Robert, 90427 Nürnberg (DE); Medlar, Donal, 91085 Weisendorf (DE); Urmoneit, Uwe, 91466 Gerhardshofen (DE)

(56) Entgegenhaltungen:
- WO-A-99/27839
- DE-A- 3 436 444
- DE-A- 19 728 788
- GB-A- 2 340 716
- US-A- 5 820 553

## Beschreibung

Die Erfindung betrifft ein Verfahren zur wiederholt gleichen Relativpositionierung eines Patienten zu einer bildgebenden diagnostischen Anlage und einer strahlentherapeutischen Anlage in Bezug auf das Koordinatensystem der jeweiligen Anlage.

Des Weiteren betrifft die Erfindung eine bildgebende diagnostische und strahlentherapeutische Anlage mit einer Positioniervorrichtung zur Relativpositionierung von Patient und Anlage, mit mindestens einer Kamera, vorzugsweise im sichtbaren und/oder infraroten Wellenlängenbereich, zur Aufnahme und Positionskontrolle eines zu behandelnden Patienten, mit einem Speichermittel zur Abspeicherung der Aufnahmen und mit einem Wiedergabemittel zur Darstellung dieser Aufnahmen.

Bei der wiederholten Herstellung von diagnostischen Bildern, wie beispielsweise bei der Röntgenbildgebung, Computertomographie, Magnetresonanztomographie, Ultraschalltomographie oder Positronenemissionstomographie oder auch bei therapeutischen, insbesondere strahlentherapeutischen Anlagen, ist es notwendig, zur Verlaufskontrolle beziehungsweise zur optimalen Behandlung des bestimmten Körperbereichs, eine im Wiederholungsfall möglichst genaue Positionierung des Patienten relativ zum Koordinatensystem der jeweiligen Anlage zu erreichen.

Bekannt ist es hierbei, den Patienten mit Markierungen zu versehen, die mit Referenzpunkten der Anlage verglichen werden, wobei Patient und Anlage derart relativ zueinander verschoben werden können, dass diese Markierungen jeweils mit einem Referenzsystem der Anlage zur Deckung gebracht werden.

Bezüglich solcher Anlagen und eines solchen Verfahrens werden auf die Offenlegungsschrift DE 44 32 890 und die Patentschrift US 6,279,579 B1 verwiesen.

Problematisch ist bei diesen bekannten Verfahren und Vorrichtungen, dass bei wiederholten Sitzungen die entsprechenden Markierungen neu am Patienten anzubringen sind, da es in der Praxis nicht durchführbar ist, den Patienten mit bleibenden Markierungen oder sichtbaren Läsionen zu versehen, die über einen langen Zeitraum unverändert bleiben.

Das neue Anbringen solcher Markierungen ist mit Fehlern behaftet, die in der Praxis dazu führen, dass Repositionierungen von Patienten sich in einem Bereich von 5-8 mm Wiederholungsgenauigkeit mit gelegentlich höheren Abweichungen bis zu 10 mm bewegten.

Aus der US 5,820,553 ist es bekannt, Positionierungsdaten vor einer Bestrahlungssitzung, beispielsweise mittels einer Röntgenvorrichtung, zu akquirieren. Die Positionierungsdaten können dann für die Erstellung des Bestrahlungsplans verwendet werden.

In der DE 34 36 444 A1 wird bereits ein Verfahren zur reproduzierbaren dreidimensionalen Positionierung eines Patienten, insbesondere zur Bestrahlung, offenbart, welches ohne Anbringen von Positionsmarkierungen auskommt und die Repositionierung des Patienten mit einer höheren Wiederholungsgenauigkeit durchgeführt werden kann.

Die DE 34 36 444 A1 offenbart dabei ein Verfahren zur wiederholt gleichen Relativpositionierung eines Patienten zu einer bildgebenden diagnostischen und/oder strahlentherapeutischen Anlage in Bezug auf das Koordinatensystem der jeweiligen Anlage, wobei
- von dem Patienten oder einem Teil des Patienten in einer ersten Sitzung mindestens zwei Referenzaufnahmen in mindestens zwei unterschiedlichen Kamerapositionen und unterschiedlichen Aufnahmeachsen gefertigt und gespeichert werden,
- in einer weiteren Sitzung aktuelle Aufnahmen bei gleicher Kameraeinstellung gefertigt werden, wobei mindestens zwei aktuelle Aufnahmen mit früheren Referenzaufnahmen aus gleicher Kameraeinstellung überlagert werden, so dass Unterschiede zwischen den Aufnahmen erkennbar werden und
- anschließend die Position des Patienten relativ zur Anlage und/oder einer diagnostischen oder therapeutischen Teilvorrichtung der Anlage so lange verändert wird, bis die Unterschiede zwischen den Referenzaufnahmen und den jeweils zur neuen Position des Patienten aktuellen Aufnahmen minimiert sind.

Hierzu werden mindestens zwei Bezugsbilder von einem Patienten in einer Bezugsposition in zwei Bildebenen und zwei aktuelle Bilder in einer neuen Position aufgenommen. Die neue Position des Patienten wird solange verändert, bis die aktuellen Bilder mit den Bezugsbildern übereinstimmen. Nachteil an diesem Verfahren ist, dass sowohl die Bezugsbilder als auch die aktuellen Bilder auf nur einer diagnostischen oder therapeutischen Anlage erstellt werden.
Es ist daher Aufgabe der Erfindung, ein einfaches Verfahren und eine einfache Vorrichtung zu finden, welche eine wiederholte Positionierung eines Patienten unterschiedlichen Anlagen erlaubt, deren Wiederholbarkeit wesentlich besser als die oben genannten 5-8 mm Positioniergenauigkeit ist.

Diese Aufgabe wird gemäß der Erfindung durch die Merkmale der beiden unabhängigen Ansprüche gelöst. Vorteilhafte Ausbildungen sind in den Unteransprüchen angegeben.
Die Erfinder haben erkannt, dass es möglich ist, die Positioniergenauigkeit eines Patienten in einer diagnostischen oder therapeutischen Anlage für wiederholte Positionierungen wesentlich zu verbessern, wenn bei einer ersten Sitzung die Lage des Patienten durch Aufnahmen in zwei voneinander unabhängigen Ebenen beziehungsweise in zwei voneinander unabhängigen Achsen gespeichert werden. Bei einer zu wiederholenden Positionierung dieses Patienten in gleicher Lage im Koordinatensystem einer anderen Anlage wird ein Vergleich der ursprünglich gewonnenen Referenzaufnahmen mit aktuellen Aufnahmen durch Kameras in gleicher Positionsausrichtung und Perspektive durchgeführt. Werden hierbei die aktuell gewonnenen Aufnahmen mit den ursprünglich hinterlegten Referenzaufnahmen überlagert, so tritt der Unterschied zwischen der ursprünglichen Lagerung und der jetzt aktuellen Lagerung des Patienten optisch zu Tage.

Besonders günstig hat sich hierbei das an sich bekannte Subtraktionsverfahren erwiesen, bei dem von einem Hintergrundbild, hier die Referenzaufnahme, die aktuelle Aufnahme pixelweise subtrahiert wird, so dass in dem, aus dieser Operation resultierenden Differenzbild der Unterschied zwischen beiden Aufnahmen sehr deutlich zu Tage tritt. Legt man ein solches Bild auf den Monitor des Operators einer Anlage, so ist es für diesen sehr leicht, den Patienten so lange zu verschieben, bis eine optimale Deckung zwischen der ursprünglichen Lage des Patienten bei der ersten Sitzung und der jetzt aktuellen Lage besteht.

Ein solches Verfahren zur Positionierung eines Patienten lässt sich auch auf einfache Weise automatisieren. So kann beispielsweise das an sich bekannte Verfahren zur Detektion von Objektbewegungen, wie es beispielsweise aus der Offenlegungsschrift DE 196 21 612 A1 bekannt ist, in seinen Grundzügen verwendet werden. Hierbei muss lediglich die erkannte Bewegung eines Objektes zwischen zwei Bildern dahingehend interpretiert werden, dass zur Erzeugung einer Deckung der aktuellen Position eines Patienten mit einer ursprünglichen Referenzposition diese erkannte "Bewegung" rückgängig gemacht wird, indem eine entsprechende Steuerung auf eine Positioniervorrichtung des Patienten entsprechend einwirkt.

Um den Patienten im dreidimensionalen Raum optimal zu repositionieren ist es notwendig, Aufnahmen in zumindest zwei voneinander unabhängigen Achsen, vorzugsweise in orthogonalen Achsen mit ursprünglich gefertigten Referenzaufnahmen mit gleicher Kameraeinstellung, zu vergleichen.

Entsprechend einem zusätzlichen Aspekt der Erfindung besteht neben der exakten Repositionierung eines Patienten auch die Möglichkeit, mit diesem Verfahren eine verbesserte Korrelation zwischen diagnostischen Aufnahmen, vorzugsweise Schichtaufnahmen, einer bildgebenden Anlage, wie zum Beispiel einem Computertomograph, und der tatsächlichen Position eines Patienten zu erreichen. Hierzu werden die visuellen Referenzaufnahmen mit ursprünglichen diagnostischen Aufnahmen, vorzugsweise Schichtaufnahmen, in Korrelation gesetzt und anschließend diese Korrelation auf eine therapeutische Anlage übertragen, so dass es nun in der therapeutischen Anlage möglich ist, eine wesentlich genauere Behandlung erkrankter Körperregionen zu erreichen. Hierdurch wird beispielsweise bei der Anwendung von Strahlentherapien mit ionisierender Strahlung sichergestellt, dass eine ausreichend hohe therapeutische Dosis in erkrankte Bereiche verabreicht wird, während die umgebenden nicht erkrankten Bereiche einer wesentlich geringeren Strahlenbelastung ausgesetzt sind und damit nicht oder nur gering geschädigt werden. Die hierfür notwendige Vorgehensweise ist allgemein bekannt, wobei der Erfolg aber von einer genauen Positionierung des zu behandelnden Gebietes abhängt.

Aufgrund der oben dargestellten Erkenntnis der Erfinder schlagen diese vor, das an sich bekannte Verfahren zur wiederholt gleichen Relativpositionierung eines Patienten zu einer bildgebenden diagnostischen Anlage und einer strahlentherapeutischen Anlage in Bezug auf das Koordinatensystem der jeweiligen Anlage dahingehend zu verbessern, dass
- von dem Patienten oder einem Teil des Patienten in einer ersten Sitzung in der bildgebenden diagnostischen Anlage mindestens zwei Referenzaufnahmen in mindestens zwei unterschiedlichen Kamerapositionen und unterschiedlichen Aufnahmeachsen gefertigt und gespeichert werden mit einer Kameraeinstellung relativ zum Koordinatensystem der bildgebenden diagnostischen Anlage,
- in einer weiteren Sitzung in der strahlentherapeutischen Anlage aktuelle Aufnahmen aus gleicher Kameraeinstellung relativ zum Koordinatensystem der strahlentherapeutischen Anlage gefertigt werden, wobei
- mindestens zwei aktuelle Aufnahmen mit früheren Referenzaufnahmen aus gleicher Kameraeinstellung überlagert werden, so dass Unterschiede zwischen den Aufnahmen erkennbar werden und
- anschließend die Position des Patienten relativ zur strahlentherapeutischen Anlage so lange verändert wird, bis die Unterschiede zwischen den Referenzaufnahmen und den jeweils zur neuen Position des Patienten aktuellen Aufnahmen minimiert sind, und
- vor der Überlagerung der Referenzaufnahmen und aktuellen Aufnahmen die Konturen aus den Aufnahmen extrahiert werden, und
- zu mindestens einer Referenzaufnahme die Position und/oder Brennweite der Kamera und/oder Aufnahmeachse relativ zur bildgebenden diagnostischen Anlage gespeichert wird und vor der Erstellung aktueller Aufnahmen die mindestens eine Kamera entsprechend den gespeicherten Daten eingerichtet wird.

Beispielsweise ergeben sich durch dieses Verfahren Vorteile bezüglich einer genaueren Therapieplanungsmöglichkeit. Es können Bildinformationen von einer diagnostischen Anlage zur therapeutischen Anlage übertragen werden. Und zur Kontrolle des Therapieerfolgs kann eine Rückführung der Informationen von der therapeutischen Anlage zur diagnostischen Anlage erfolgen. Weiterhin besteht auch die Möglichkeit, eine erste Referenzaufnahme zur genauen Positionierung des Patienten in einem therapeutischen System zu fertigen und anschließend zur Therapieüberwachung diese Aufnahme für die spätere Positionierung des Patienten im diagnostischen System zu verwenden.

Besonders eignet sich für das oben geschilderte Verfahren eine Videoanlage, die durch entsprechend schnelle und effektive Rechenprozesse Differenzbilder der aktuellen Videokamera mit den Referenzaufnahmen der ersten Sitzung vergleicht.

Es ist in diesem Zusammenhang darauf hinzuweisen, dass es möglich ist, Kameraeinstellungen mit unterschiedlicher Brennweite zu verwenden, jedoch ist es notwendig, die gleiche Perspektive zu erhalten.

Neben der visuellen Kontrolle der überlagerten Bilder beziehungsweise Differenzbilder auf einem Kontrollmonitor ist es auch möglich, rein rechnerische Informationen über die Unterschiede der Referenzaufnahmen mit den aktuellen Aufnahmen in numerischer oder sonstiger adäquater Form anzuzeigen.

Besonders vorteilhaft hat sich für die Darstellung der Unterschiede zwischen Referenzaufnahme und aktueller Aufnahme das an sich bekannte Subtraktionsverfahren erwiesen, welches beispielsweise zur automatischen Detektion von Bewegungen in Raumüberwachungsvideosystemen bekannt ist. Neben diesem Subtraktionsverfahren besteht auch die Möglichkeit ein Additionsverfahren oder ein Mischverfahren zu verwenden, welches die Differenzen der Aufnahmen hervorhebt.

Erfindungsgemäß besteht auch die Möglichkeit die errechneten Differenzen in den Aufnahmen farbig hervorzuheben und in die aktuellen Aufnahmen einzumischen. Soll eine besonders genaue Positionierung vorgenommen werden, so können ebenfalls einzelne Teilbereiche vergrößert dargestellt werden, damit auch kleine Unterschiede am Monitor für das Bedienpersonal besonders gut erkennbar werden.

Eine weitere Möglichkeit die Differenzen zwischen den Referenzbildern und aktuellen Bildern stärker hervortreten zu lassen besteht auch in einer zusätzlichen Bildverarbeitung der Referenzbilder und der aktuellen Bilder, beispielsweise durch eine Erhöhung der Gradation oder eine Hervorhebung von Konturen.

Zu mindestens einer Referenzaufnahme wird die Position und/oder Perspektive der Kamera und/oder Aufnahmeachse relativ zur bildgebenden diagnostischen und/oder therapeutischen Anlage gespeichert und vor der Erstellung aktueller Aufnahmen die mindestens eine Kamera entsprechend den gespeicherten Daten eingerichtet. Hierdurch wird es möglich, die Referenzaufnahmen zwischen verschiedenen Anlagen zu übertragen, so dass beispielsweise Aufnahmen eines Patienten in einem Röntgen-Computer-Tomographen besser mit Aufnahmen durch ein anderes bildgebendes Verfahren, beispielsweise eines Kernspin-Tomographen, verglichen werden können. Hierbei sind beliebige Kombinationen bekannter diagnostischer bildgebender Anlagen möglich.

Erfindungsgemäß schlagen die Erfinder außerdem vor, das Verfahren dahingehend zu erweitern, dass die Referenzaufnahmen der ersten Anlage mit diagnostischen Schichtaufnahmen dieser Anlage räumlich in Korrelation gebracht werden und in der zweiten Anlage wieder die räumliche Korrelation der diagnostischen Schichtaufnahmen zur aktuellen Aufnahme hergestellt wird, um die Positionierung des Patienten in der strahlentherapeutischen Anlage zu verbessern und einen zu behandelnden Krankheitsherd gezielter zu behandeln.

Vorteilhaft besteht außerdem die Möglichkeit, die gewonnenen Referenzaufnahmen einschließlich der hiermit korrelierten Kameraeinstellungen mit Hilfe von Datenfernverbindung an eine weitere Anlage zu übertragen, wobei die weitere Anlage ihre mindestens eine Kamera, vorzugsweise zwei Kameras, automatisch entsprechend den übermittelten Kameraeinstellungen der ersten Anlage justiert.

Neben dem erfindungsgemäßen Verfahren schlagen die Erfinder auch vor, die an sich bekannte bildgebende diagnostische Anlage und strahlentherapeutische Anlage mit einer Positioniervorrichtung zur Relativpositionierung von Patient innerhalb des Koordinatensystems der Anlage, mit mindestens einer Kamera, im sichtbaren und/oder infraroten Wellenlängenbereich, zur Positionskontrolle eines zu behandelnden Patienten und zur Erstellung von mindestens zwei Aufnahmen in mindestens zwei unabhängigen Aufnahmeachsen, mit Speichermittel zur Abspeicherung der Aufnahmen und mit einem Monitor zur Darstellung dieser Aufnahmen, dahingehend weiterzuentwickeln, dass
- Mittel zum Vergleich von aktuell erstellten Aufnahmen mit in einer früheren Sitzung erstellten und gespeicherten Referenzaufnahmen und
- Mittel zur Darstellung eines Unterschiedes zwischen Referenzaufnahmen und aktuellen Aufnahmen vorhanden sind, wobei die Aufnahme der Referenzaufnahmen in der bildgebenden diagnostischen Anlage und die Aufnahme der aktuellen Aufnahmen in der strahlentherapeutischen Anlage stattfinden, und
- das Mittel zum Vergleich der aktuellen Aufnahmen mit den Referenzaufnahmen ein Computersystem mit einem Computerprogramm zur Durchführung des Verfahrens gemäß einem der Ansprüche des Verfahrens und zur Darstellung von Bildunterschieden auf dem Monitor aufweist, wobei die mindestens eine Kamera zu Erzeugung der Referenzaufnahmen und aktuellen Aufnahmen im Raum und/oder in ihrer Orientierung beweglich, vorzugsweise automatisch gesteuert, angebracht ist.

Durch eine derart gestaltete diagnostische oder therapeutische Anlage ist es nun möglich, das zuvor geschilderte Verfahren durchzuführen und damit eine verbesserte Repositionierung der Patientenlage im Koordinatensystem der Anlage zu erreichen, da das Bedienpersonal nun aufgrund der mindestens zwei Aufnahmen in der Lage ist, Abweichungen der aktuellen Lage des Patienten in Relation zu einer zuvor definierten Referenzlage zu bestimmen und ihn solange zu bewegen, bis die Unterschiede in den aktuellen Aufnahmen und Referenzaufnahmen auf ein Minimum reduziert sind.

Es ergeben sich hierdurch Vorteile bezüglich einer genaueren Therapieplanungsmöglichkeit, indem zum Beispiel die Bildinformationen von einer diagnostischen Anlage zur therapeutischen Anlage übertragen werden. Schließlich wird nochmals die verbesserte Therapieerfolgskontrolle durch die Rückführung der Informationen von der therapeutischen Anlage zur diagnostischen Anlage übertragen. Weiterhin besteht auch die Möglichkeit, eine erste Referenzaufnahme zur genauen Positionierung des Patienten in einem therapeutischen System zu fertigen und anschließend zur Therapieüberwachung diese Aufnahme für die spätere Positionierung des Patienten im diagnostischen System zu verwenden.

Ergänzend sei hier noch erwähnt, dass es sich bei den oben genannten Koordinatensystemen der Anlage nicht unbedingt um ein ortsfestes Koordinatensystem handeln muss, welches mit dem gebäudeverbundenen Teil der therapeutischen oder diagnostischen Anlage identisch ist, sondern dass es sich auch um das Koordinatensystem der bildgebenden Aufnahmevorrichtung beziehungsweise um das Koordinatensystem des therapeutisch wirkenden Teils einer Strahlenquelle handeln kann.

Obwohl es durchaus möglich ist, die Mittel zum Vergleich der aktuellen Aufnahmen mit den Referenzaufnahmen rein hardwaremäßig auszuführen, so ist es doch heutzutage von Vorteil, hierzu ein Computersystem mit einem Computerprogramm vorzusehen, welches die Verfahrensschritte des erfindungsgemäßen Verfahrens durchführt und Bildunterschiede auf einem Monitor darstellt.

Eine weitere vorteilhafte Ausführung der erfindungsgemäßen Anlage kann eine Sende- und/oder Empfangsvorrichtung zu einem Datennetz für die Übermittlung von Bildern und gegebenenfalls deren Kameraeinstellungen vorsehen. Hierdurch wird es möglich, eine Referenzposition eines Patienten, die in einer ersten Anlage gewonnen wird an eine zweite Anlage auf einfache Weise zu übertragen. Beispielsweise kann hierzu eine direkte Datenverbindung hergestellt oder die Übertragung kann über das Internet, vorzugsweise mit entsprechenden Sicherheitsvorrichtungen, übertragen werden.

Bei der oben genannten Möglichkeit handelt es sich grundsätzlich um eine komplette Übertragung der aufgenommenen Referenzaufnahmen und gegebenenfalls Kameraeinstellungen, wobei jedoch auch Methoden zur Verfügung stehen, zwei ähnliche Dateien über eine Datennetzverbindung zu vergleichen, ohne dass die vollständigen Datensätze auf einem einzigen Computer vorliegen müssen. Bezüglich dieser Methode wird auf die internationale Patentanmeldung WO 00/04467 verwiesen, deren Inhalt bezüglich des Vergleiches zweier Datensätze über eine Netzwerkverbindung vollinhaltlich in den Offenbarungsgehalt dieser Anmeldung übernommen wird. In dieser Anmeldung wird ein System und ein Verfahren vorgestellt, mit denen es möglich ist, zwei ähnliche Dateien, beispielsweise eine Referenzaufnahme und eine aktuelle Aufnahme, auf zwei entfernt voneinander stehenden und mit einer Datenverbindung verbundenen Computern "online" zu vergleichen, ohne dass die vollständigen Dateien zwischen den Computern ausgetauscht werden müssen. Bei dieser Art der Datenübertragung entsteht ein zusätzlicher Sicherheitsaspekt, da über das Datennetz keine vollständigen digitalisierten Aufnahmen übertragen werden und somit durch das unerlaubte Abhören einer solchen Verbindung keine Möglichkeit besteht, die eigentlichen Bilder zu erkennen, ohne dass die Referenzdatei vorliegt.

Die mindestens eine Kamera ist zu Erzeugung der Referenzaufnahmen und aktuellen Aufnahmen im Raum und/oder Orientierung beweglich, vorzugsweise automatisch gesteuert, angebracht. Durch diese Variabilität einer Anlage besteht die Möglichkeit sich auf die Gegebenheiten und Kameraeinstellungen von Referenzaufnahmen beliebig anzupassen, so dass eine möglichst hohe Kompatibilität zwischen verschiedenen diagnostischen und therapeutischen Anlagen erreicht werden kann.

Vorzugsweise schlagen die Erfinder vor, dass mindestens zwei Kameras zur Verfügung stehen sollten, welche gleichzeitig aus unterschiedlichen Kameraeinstellungen heraus die Referenzaufnahmen und/oder aktuellen Aufnahmen erstellen, wobei diese in voneinander unabhängig Achsen, vorzugsweise orthogonal zueinander angeordnet sind.

Zusätzliche Merkmale und Vorteile der Erfindung ergeben sich aus der nachfolgenden Beschreibung bevorzugter Ausführungsbeispiele unter Bezugnahme auf die Zeichnungen.

Die Ausführungsbeispiele sollen nachfolgend anhand der Zeichnungen näher erläutert werden. Es stellen im Einzelnen dar:
- Figur 1:: Subtraktionsverfahren zweier visueller Aufnahmen eines Patienten in unterschiedlichen Positionen und der sich daraus ergebenden Subtraktionsaufnahme;
- Figur 2:: Computertomograph mit zwei Kameras zur Erstellung einer Referenzaufnahme;
- Figur 3:: Schematische Darstellung einer Repositionierung in einem CT;
- Figur 4:: Repositionierung eines Patienten in einer strahlentherapeutischen Anlage;
- Figur 5:: Schematische Darstellung der erfindungsgemäßen Repositionierung in einem Computertomograph;
- Figur 6:: Schematische Darstellung einer Therapieplanung und Therapierkontrolle mit Hilfe der erfindungsgemäßen Patientenpositionierung.

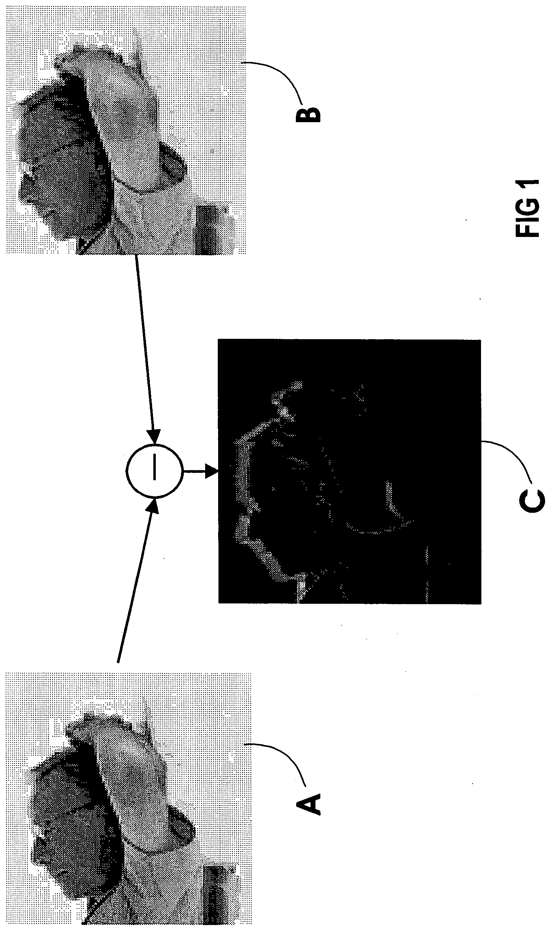

Die Figur 1 zeigt das grundsätzliche Prinzip des erfindungsgemäßen Verfahrens zur wiederholten Positionierung eines Patienten in einer Anlage. Die Aufnahme A zeigt einen Patienten in einer ersten Referenzposition, wie sie beispielsweise in einem Computertomograph durch eine Videokamera aufgenommen wurde.

Das Bild B zeigt den gleichen Patienten in der gleichen Anlage, aufgenommen mit identischer Kameraposition, Perspektive und Aufnahmeachse, jedoch besteht eine Differenz in der Lage des Patienten zur ursprünglichen Aufnahme in Bezug auf das Koordinatensystem des Computertomographs. Beide Aufnahmen werden in einem Bildverarbeitungssystem überlagert, wobei hier ein Subtraktionsverfahren angewendet wird, bei dem pixelweise die Bildinformationen des Bildes A von den Bildinformationen des Bildes B abgezogen werden, so dass im Ergebnis das in der Mitte dargestellte Bild C resultiert. Handelt es sich bei den beiden Aufnahmen A und B um identische Aufnahmen, das heißt Patienten in identischer Position bei gegebener Kameraeinstellung, so ergibt sich auf dem Subtraktionsbild C eine schwarze homogene Fläche. Sind die beiden Aufnahmen A und B jedoch unterschiedlich, so werden die Unterschiede - wie in diesem Beispiel gezeigt - durch helle Schattierungen hervorgehoben. In der dargestellten Aufnahme ist deutlich zu erkennen, dass der Kopf des Patienten in der zweiten Aufnahme B eine höhere Position einnimmt, als in der ersten Aufnahme, wodurch die Kontur des Gesichtes im Frontbereich als heller Schatten auffällt.

Die in der Figur 1 gezeigten Aufnahmen in der Querachse lassen Verschiebungen in der Körperlängsachse und der sagittalen Achse hervortreten, während Aufnahmen in sagittaler Richtung beziehungsweise in der Körperlängsachse eine Positionierung in der Längsachse und der Querachse des Patienten ermöglichen.

Auf diese Weise sind kleinste Abweichungen von einer einmal in einem Referenzbild A definierten Position bestens erkennbar und somit auch korrigierbar.

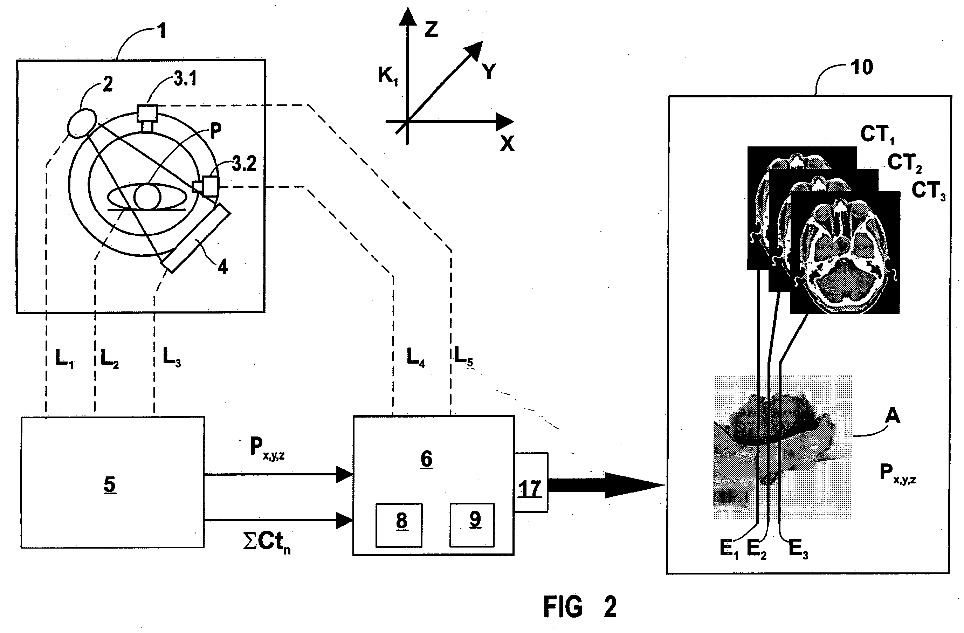

In der Figur 2 wird ein erfindungsgemäßer Computertomograph 1 dargestellt, welcher in bekannter Weise über eine Röntgenröhre 2 mit einem Detektor 4 verfügt, mit denen sich Schnittbilder CT₁ bis CTₙ eines Patienten P erstellen lassen. Zusätzlich verfügt das CT über zwei Videokameras 3.1 und 3.2, welche in der Z- und X-Achse des Koordinatensystems K₁ des CT's angeordnet sind. Die Röntgenröhre 2 und der Detektor 4 sind mit einem CT-Steuer-Bildrechner über die Leitungen L₁ und L₃ verbunden, während die Positionierung des Patienten P über die Leitung L₂ mit Hilfe der Rechnereinheit 5 ebenfalls vorgenommen werden kann. Sowohl die vom Rechner 5 ermittelten Positionsdaten P_{x,y,z}, als auch die CT-Bildinformationen CTₙ werden an das Videoaufnahmesystem 6 übermittelt. Das Videoaufnahmesystem 6 erhält außerdem die Bildinformationen der beiden Videokameras 3.1 und 3.2 in den Achsen Z und X. Somit kann vom Videoaufnahmesystem eine Korrelation zwischen den errechneten CT-Schichtaufnahmen CT₁ bis CTₙ in Relation zur visuellen Aufnahme in den Ebenen E₁ bis Eₙ hergestellt werden.

In der Figur 2 ist aus Gründen der Übersichtlichkeit nur die Aufnahme der Kamera 3.2 mit dem entsprechenden Videobild A dargestellt. Durch dieses Bild sind beispielhaft drei Ebenen E₁ bis E₃ angezeigt, die mit den verbundenen CT-Aufnahmen CT₁ bis CT₃ korreliert sind.

Ergänzend sei hierbei erwähnt, dass das Videoaufnahmesystem selbstverständlich über ein Display 8 verfügt, auf dem die entsprechenden Informationen des Kastens 10 dargestellt werden können. Des Weiteren ist ein Speicher 9 vorgesehen, der die gesammelten Bilddaten speichert.

Durch das oben beschriebene System lässt sich also eine genaue Position eines Patienten durch die Speicherung von Videoaufnahmen und gegebenenfalls damit verbundener ortspezifischer diagnostischer Informationen, wie beispielsweise CT-Aufnahmen oder Kernspinaufnahmen, in Korrelation setzen. Sind diese Aufnahmen abrufbar gespeichert, so besteht jederzeit wieder die Möglichkeit den Patienten bei einer wiederholten Untersuchung in die entsprechende Lage zu repositionieren. Ebenso können die im Informationskasten 10 dargestellten Informationen - CT-Bilder, Referenzaufnahmen, Positionsdaten, gegebenenfalls Kameraeinstellungen - mit der Sendevorrichtung 17 ausgegeben und beispielsweise an eine andere Anlage übertragen werden. Eine Sendevorrichtung im Sinne dieser Erfindung ist beispielsweise auch ein Internet-Anschluss oder ein sonstiger Anschluss an eine Telekommunikationsverbindung oder ein Netzwerk.

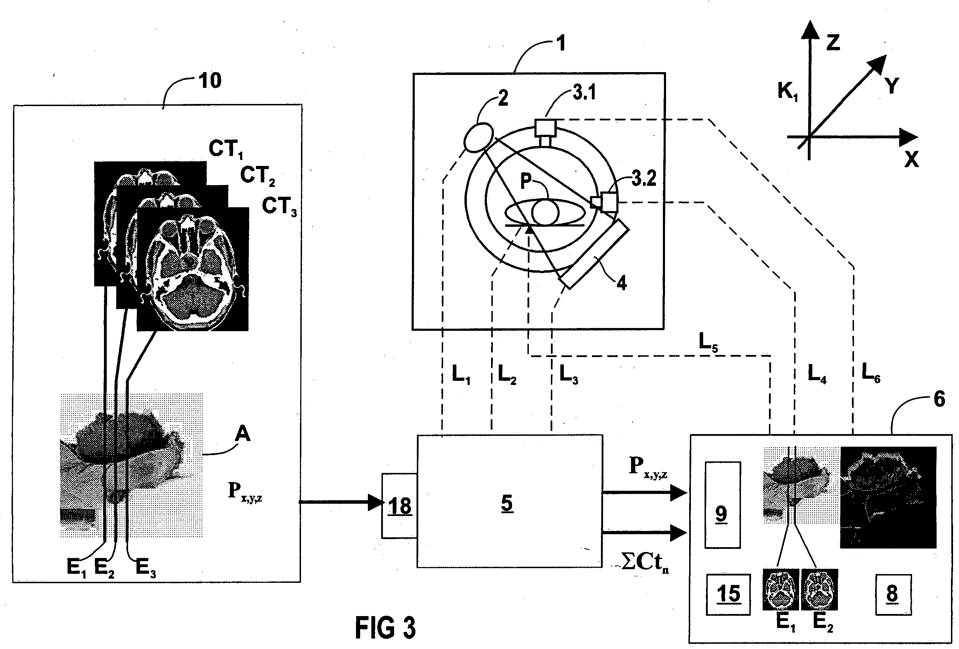

Eine solche Repositionierung ist in der Figur 3 dargestellt. Hier wird ein CT₁ mit Röntgenröhre 2, Detektor 4 und zwei Videokameras 3.1 und 3.2 gezeigt, welche identisch zum CT aus Figur 2 angeordnet sind. Die gesamten Bild- und Positionsinformationen die in einer ersten Sitzung im CT aus Figur 2 gewonnen wurden - dargestellt durch den Kasten 10 -, werden dem CT in Figur 3 über eine Empfangsvorrichtung 18 zur Verfügung gestellt. Das Bedienpersonal hat nun die Möglichkeit im Videoaufnahmesystem 6 die aktuell erstellten Aufnahmen B durch die Kameras 3.1 und 3.2 mit den entsprechenden Referenzaufnahmen A einer früheren Sitzung zu vergleichen und das Resultat des Vergleichs, beispielsweise durch eine Subtraktionsaufnahme C, wie sie aus Figur 1 bekannt ist, auf einem Monitor 8 anzeigen zu lassen. Hierauf kann eine entsprechende Positionskorrektur, dargestellt durch die Verbindung L₆, vorgenommen werden, bis wiederum eine identische Positionierung der Patienten P vorliegt. Anschließend können wieder vergleichende CT-Aufnahmen hergestellt werden, die aufgrund der verbesserten Positionierungsgenauigkeit des Patienten nun wesentlich genauer, das heißt, bis auf eine Ortsungenauigkeit von 1 bis 2 mm, mit alten CT-Schichtaufnahmen verglichen werden können. Auf diese Weise ergibt sich eine wesentlich exaktere Verlaufsdiagnostik, als es im Stand der Technik möglich war. Zur rechnerischen Unterstützung ist ein Computer 15 Bestandteil des Videoaufnahmesystems 6 oder steht diesem zumindest zur Verfügung.

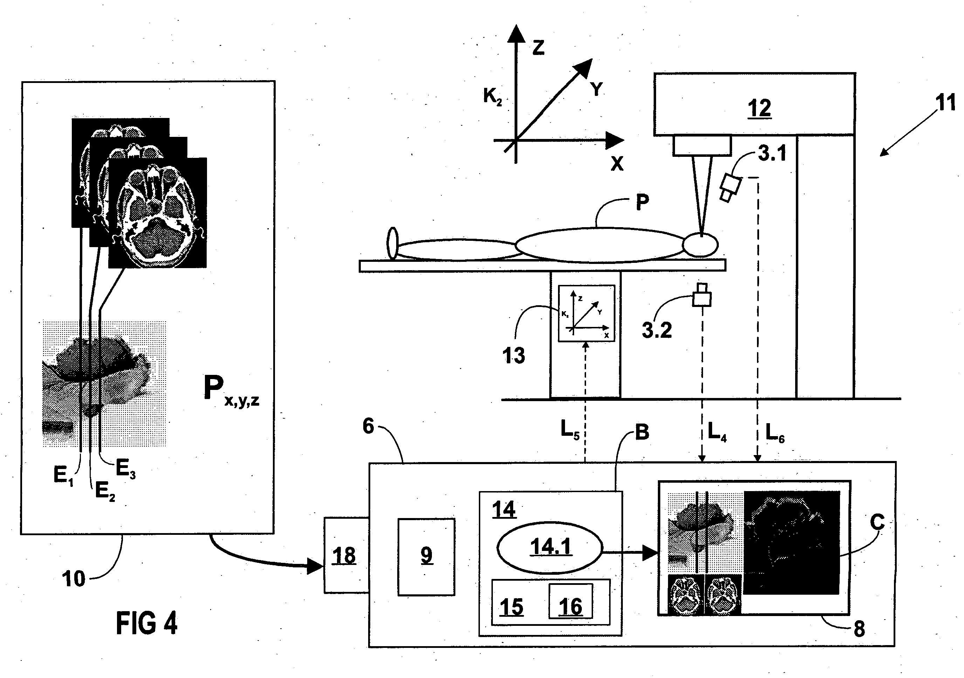

Die Figur 4 zeigt eine weitere Anwendungsmöglichkeit des erfindungsgemäßen Positionierungsverfahrens. Hier ist eine Strahlentherapieanlage 11 mit einer Strahlenquelle 12 dargestellt, mit der ein Patient P beispielsweise einer Tumorbehandlung im Kopf oder sonstigen Körperregionen unterzogen werden kann. Bei solchen Therapien ist es wesentlich, dass die Wirkung der Strahlung sich möglichst genau im Bereich des Tumors manifestiert, während umgebende und gesunde Bereiche nicht betroffen werden sollten, da durch eine zu hohe Strahlenbelastung gesunder Bereiche diese wiederum zur Karzinombildung angeregt oder auch in sonstiger unerwünschter Weise geschädigt werden könnten. Es ist daher wesentlich, die genaue Position eines Tumors in einem betrachteten Bereich zu kennen, damit die Strahlentherapie zielgenau angewendet werden kann.

Erfindungsgemäß erhält nun die Strahlentherapieanlage 11 die Bild- und Positionsinformationen ursprünglicher Referenzaufnahmen, beispielsweise durch das CT aus Figur 1. Im Videoaufnahmensystem 6 der Strahlentherapieanlage 11 kann nun - wie zuvor geschildert - durch die Erzeugung eines Bildvergleiches und die Erstellung eines Differenzbildes C, welches auf einem Monitor 8 betrachtbar ist, die Position des Patienten P im Koordinatensystem K₂ der Strahlentherapieanlage 11 solange korrigiert werden, bis die Lage des Patienten P in diesem Koordinatensystem K₂ mit der ursprünglichen Lage des Patienten P im Koordinatensystem K₁ der CT-Anlage übereinstimmt. Wie zuvor geschildert, wird diese Korrektur über eine Bildaufbereitung 14, welche über ein Computersystem 15 mit entsprechendem Programm 16 verfügt, vorzugsweise über ein Subtraktionsverfahren 14.1, vorgenommen, bei dem ein Differenzbild C zwischen einer ursprünglichen Referenzaufnahme A und einer aktuellen Aufnahme B erzeugt wird.

Hier in der Figur 4 wird beispielhaft die Referenzaufnahme A mit einer neuen Videoaufnahme B der Kamera 3.2 verglichen und das Ergebnis auf dem Monitor 8 mit der Differenzaufnahme C dargestellt. Aufgrund des starken Hervortretens von Lageveränderung in der Differenzaufnahme C kann das Bedienpersonal nun über die Positioniereinrichtung 13 die Lage des Patienten solange verändern, bis die neue Positionierung des Patienten optimiert ist.

Neben dieser manuellen Möglichkeit der Lageänderung aufgrund eines sichtbaren Bildes und der dargestellten Lageunterschiede, besteht auch die Möglichkeit einer automatischen Positionskorrektur, indem aufgrund der Bildinformation, beispielsweise der Summe der Unterschiede der Bildinformationen in einem Differenzbild, die Lage des Patienten solange verändert wird, bis diese Unterschiedssumme minimiert ist.

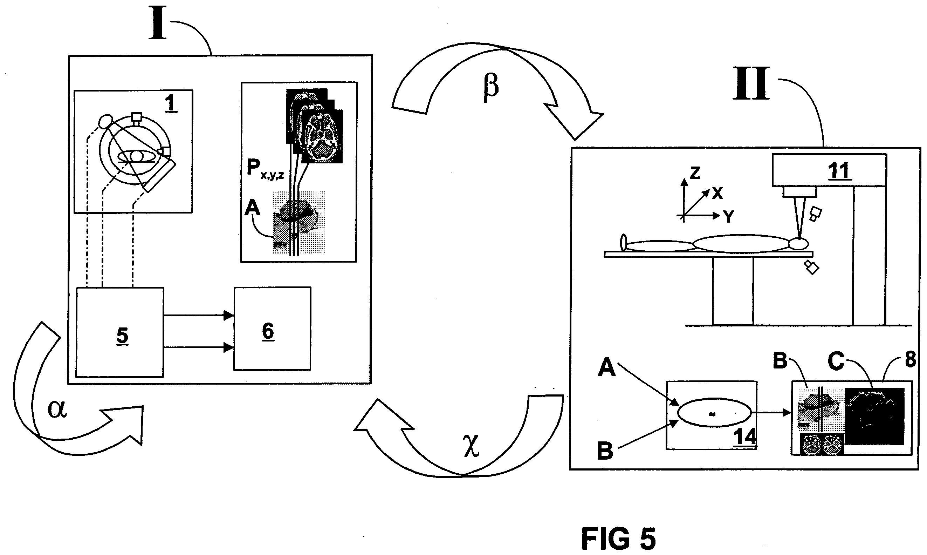

Die Figur 5 zeigt nochmals in einer Übersicht die Möglichkeiten des erfindungsgemäßen Verfahrens beziehungsweise der erfindungsgemäßen diagnostischen Anlage I und einer therapeutischen Anlage II.

Dargestellt sind die Vorteile der verbesserten Repositionierung innerhalb einer gleichen oder auch derselben diagnostischen Anlage I durch den rekursiven Pfeil α. Weiterhin die Vorteile bezüglich einer genaueren Therapieplanungsmöglichkeit durch den Pfeil β von der diagnostischen Anlage I zur therapeutischen Anlage II und schließlich nochmals die verbesserte Therapieerfolgskontrolle durch die Rückführung der Informationen von der therapeutischen Anlage II zur diagnostischen Anlage I, dargestellt durch den Pfeil y. Hierbei ist darauf hinzuweisen, dass auch die Möglichkeit besteht, eine erste Referenzaufnahme zur genauen Positionierung des Patienten in einem therapeutischen System zu fertigen und anschließend zur Therapieüberwachung diese Aufnahme für die spätere Positionierung des Patienten im diagnostischen System zu verwenden.

Zusammenfassend stellt die Erfindung also ein Verfahren zur Repositionierung eines Patienten in einer diagnostisch/therapeutischen Anlage und eine solche Anlage dar, wobei eine wiederholt genaue Repositionierung durch Erzeugung von Differenzaufnahmen aus einer Referenzaufnahme und einer aktuellen Aufnahme in je zwei unabhängigen Aufnahmeachsen und Minimierung der sichtbaren Differenzen erreicht wird. Diese sehr einfache Repositionierungsmöglichkeit eines Patienten in einer bildgebenden, diagnostischen und/oder strahlentherapeutischen Anlage mit hoher Genauigkeit kann auch für bestehende Anlagen verwendet werden. Es muss lediglich Sorge dafür getragen werden, dass in den gewünschten Korrekturebenen Kameras in exakter Position und Einstellung verfügbar sind, deren Bilder anschließend in einer Bildaufbereitungssoftware verarbeitet werden müssen. Eine zusätzliche Ausrüstung einer solchen Anlage mit einer automatischen Positionierung ist ebenfalls möglich. Aufgrund des erfindungsgemäßen Verfahrens wird damit eine wesentlich verbesserte Verlaufskontrolle, Therapieplanung und Therapiekontrolle ermöglicht.

Es versteht sich, dass die vorstehend genannten Merkmale der Erfindung nicht nur in der jeweils angegebenen Kombination, sondern auch in anderen Kombinationen oder in Alleinstellung verwendbar sind, ohne den Rahmen der Erfindung zu verlassen.

## Patentansprüche

1. Verfahren zur wiederholt gleichen Relativpositionierung eines Patienten (P) zu einer bildgebenden diagnostischen Anlage (I) und einer strahlentherapeutischen Anlage (II) in Bezug auf das Koordinatensystem (K₁, K₂) der jeweiligen Anlage (I, II), wobei
- von dem Patienten (P) oder einem Teil des Patienten (P) in einer ersten Sitzung in der bildgebende diagnostischen Anlage (I) mindestens zwei Referenzaufnahmen (A) in mindestens zwei unterschiedlichen Kamerapositionen und unterschiedlichen Aufnahmeachsen gefertigt und gespeichert werden mit einer Kameraeinstellung relativ zum Koordinatensystem (K₁) der bildgebenden diagnostischen Anlage (I),
- in einer weiteren Sitzung in der strahlentherapeutischen Anlage (II) aktuelle Aufnahmen (B) aus gleicher Kameraeinstellung relativ zum Koordinatensystem (K₂) der strahlentherapeutischen Anlage (II) gefertigt werden, wobei
- mindestens zwei aktuelle Aufnahmen (B) mit früheren Referenzaufnahmen (A) aus gleicher-Kameraeinstellung überlagert werden, so dass Unterschiede zwischen den Aufnahmen (C) erkennbar werden und
- anschließend die Position (P_{x,y,z}) des Patienten (P) relativ zur strahlentherapeutischen Anlage (II) so lange verändert wird, bis die Unterschiede (C) zwischen den Referenzaufnahmen (A) und den jeweils zur neuen Position des Patienten aktuellen Aufnahmen (B) minimiert sind, und
- vor der Überlagerung (C) der Referenzaufnahmen (A) und aktuellen Aufnahmen (B) die Konturen aus den Aufnahmen (A, B) extrahiert werden, und
- zu mindestens einer Referenzaufnahme (A) die Position und/oder Brennweite der Kamera (3.1; 3.2) und/oder Aufnahmeachse relativ zur bildgebenden diagnostischen Anlage (I) gespeichert wird und vor der Erstellung aktueller Aufnahmen (B) die mindestens eine Kamera (3.1, 3.2) entsprechend den gespeicherten Daten eingerichtet wird.

2. Verfahren nach Patentanspruch 1, **dadurch gekennzeichnet, dass** die Aufnahmen (A, B) digital gespeichert und zumindest die überlagerten Bilder der Aufnahmen (C) an einem Kontrollmonitor (8) dargestellt werden.

3. Verfahren nach Patentanspruch 1 oder Patentanspruch 2, **dadurch gekennzeichnet, dass** die Überlagerung (C) der Referenzaufnahmen (A) und aktuellen Aufnahmen (B) durch ein Subtraktionsverfahren erzeugt wird.

4. Verfahren nach einem der Patentansprüche 1 bis 3, **dadurch gekennzeichnet, dass** die Überlagerung (C) der Referenzaufnahmen (A) und aktuellen Aufnahmen (B) durch ein Additionsverfahren erzeugt wird.

5. Verfahren nach einem der Patentansprüche 1 bis 4, **dadurch gekennzeichnet, dass** die Überlagerung (C) der Referenzaufnahmen (A) und aktuellen Aufnahmen (B) durch ein Mischverfahren erzeugt wird.

6. Verfahren nach einem der Patentansprüche 1 bis 5, **dadurch gekennzeichnet, dass** vor der Überlagerung (C) der Referenzaufnahmen (A) und aktuellen Aufnahmen (B) eine Bildbearbeitung (14) zur besseren Darstellung der Bildunterschiede stattfindet.

7. Verfahren nach einem der Patentansprüche 1 bis 6, **dadurch gekennzeichnet, dass** die verwendeten Aufnahmeachsen voneinander unabhängig, vorzugsweise orthogonal zueinander, sind.

8. Verfahren nach einem der Patentansprüche 1 bis 7, **dadurch gekennzeichnet, dass** die bildgebende diagnostische Anlage (I) ein Röntgen-Computer-Tomograph (1) oder ein Magnetresonanz-Tomograph oder ein Positronenemissions-Tomograph ist.

9. Verfahren nach einem der Patentansprüche 1 bis 8, **dadurch gekennzeichnet, dass** die strahlentherapeutische Anlage (II) eine strahlentherapeutische Anlage (11) auf der Basis von ionisierender Strahlung ist.

10. Verfahren nach einem der Patentansprüche 1 bis 9, **dadurch gekennzeichnet, dass** Referenzaufnahmen (A) einschließlich der Kameraeinstellungen der bildgebenden diagnostischen Anlage (I) über eine Datenfernverbindung an die strahlentherapeutische Anlage (II) übertragen werden, wobei die strahlentherapeutische Anlage (II) ihre Kamera (3.1, 3.2) automatisch entsprechend den übermittelten Kameraeinstellungen der bildgebenden diagnostischen Anlage (I) justiert.

11. Verfahren nach einem der Patentansprüche 1 bis 10, **dadurch gekennzeichnet, dass** die Referenzaufnahmen (A) der bildgebenden diagnostischen Anlage (I) mit diagnostischen Schichtaufnahmen (CT1, CT2, CT3) dieser Anlage räumlich in Korrelation gebracht werden und in der strahlentherapeutischen Anlage (II) wieder die räumliche Korrelation der diagnostischen Schichtaufnahmen (CT1, CT2, CT3) zur aktuellen Aufnahme (B) hergestellt wird, um eine genauere Positionierung eines zu behandelnden Krankheitsherdes zu erreichen.

12. Bildgebende diagnostische Anlage (I) und strahlentherapeutische Anlage (II) mit:
- einer Positioniervorrichtung (13) zur Relativpositionierung von Patient und Anlage (I, II),
- mindestens einer Kamera (3.1, 3.2), empfindlich im sichtbaren und/oder infraroten Wellenlängenbereich, zur Positionskontrolle eines zu behandelnden Patienten und zur Erstellung von mindestens zwei Aufnahmen (A, B) in mindestens zwei unabhängigen Aufnahmeachsen,
- einem Speichermittel zur Abspeicherung der Aufnahmen (A) und
- einem Monitor (8) zur Darstellung dieser Aufnahmen, wobei
- Mittel zum Vergleich (14) von aktuell erstellten Aufnahmen (B) mit in einer früheren Sitzung erstellten und gespeicherten Referenzaufnahmen (A) und
- Mittel zur Darstellung eines Unterschiedes (8) zwischen Referenzaufnahmen (A) und aktuellen Aufnahmen (B) vorhanden sind, wobei die Aufnahme der Referenzaufnahmen (A) in der bildgebenden diagnostischen Anlage (I) und die Aufnahme der aktuellen Aufnahmen (B) in der strahlentherapeutischen Anlage (II) stattfinden, und
- das Mittel (14) zum Vergleich der aktuellen Aufnahmen (B) mit den Referenzaufnahmen (A) ein Computersystem (15) mit einem Computerprogramm zur Durchführung des Verfahrens gemäß einem der Ansprüche 1 bis 11 und zur Darstellung von Bildunterschieden (C) auf dem Monitor (8) aufweist, wobei die mindestens eine Kamera zu Erzeugung der Referenzaufnahmen (A) und aktuellen Aufnahmen (B) im Raum und/oder in ihrer Orientierung beweglich, vorzugsweise automatisch gesteuert, angebracht ist.

13. Bildgebende diagnostische Anlage (I) und strahlentherapeutische Anlage (II) nach Patentanspruch 12, **dadurch gekennzeichnet, dass** Sende- und/oder Empfangsvorrichtungen (17, 18) zu einem Datennetz für übermittelte Bilder (A) und gegebenenfalls deren Kameraeinstellungen vorgesehen sind.

14. Bildgebende diagnostische Anlage (I) und strahlentherapeutische Anlage (II) nach einem der Patentansprüche 12 bis 13, **dadurch gekennzeichnet, dass** mindestens zwei Kameras (3.1, 3.2) zu Erzeugung der Referenzaufnahmen (A) und/oder aktuellen Aufnahmen (B) angebracht sind, wobei deren Aufnahmeachsen unabhängig voneinander, vorzugsweise orthogonal zueinander, angeordnet sind.

## Claims

1. Method for repeated relative positioning of a patient (P) with respect to a diagnostic imaging installation (I) and a radiotherapy installation (II) in relation to the coordinate system (K₁, K₂) of the respective installation (I, II), wherein
- at least two reference recordings (A) of the patient (P) or a part of the patient (P) in at least two different camera positions and different recording axes are produced and stored in a first session in the diagnostic imaging installation (I) with a camera setting relative to the coordinate system (K₁) of the diagnostic imaging installation (I),
- in a further session in the radiotherapy installation (II), current recordings (B) are produced from the same camera setting relative to the coordinate system (K₂) of the radiotherapy installation (II), wherein
- at least two current recordings (B) are superimposed with earlier reference recordings (A) from the same camera setting, so that differences between the recordings (C) can be identified and
- then the position (P_{x,y},_{z}) of the patient (P) relative to the radiotherapy installation (II) is modified until the differences (C) between the reference recordings (A) and the current recordings (B) in each case with respect to the new position of the patient are minimised, and
- before superimposing (C) the reference recordings (A) and current recordings (B), the contours are extracted from the recordings (A, B), and
- with respect to at least one reference recording (A) the position and/or focal width of the camera (3.1, 3.2) and/or recording axis relative to the diagnostic imaging installation (I) is stored and prior to creating current recordings (B) the at least one camera (3.1, 3.2) is set up according to the stored data.

2. Method according to claim 1, **characterised in that** the recordings (A, B) are stored digitally and at least the superimposed images of the recordings (C) are displayed on a control monitor (8).

3. Method according to claim 1 or claim 2, **characterised in that** the superimposition (C) of the reference recordings (A) and current recordings (B) is generated by a subtraction method.

4. Method according to one of claims 1 to 3, **characterised in that** the superimposition (C) of the reference recordings (A) and current recordings (B) is generated by an addition method.

5. Method according to one of claims 1 to 4, **characterised in that** the superimposition (C) of the reference recordings (A) and current recordings (B) is generated by a mixed method.

6. Method according to one of claims 1 to 5, **characterised in that** before superimposing (C) the reference recordings (A) and current recordings (B), image processing (14) is performed for improved representation of the image differences.

7. Method according to one of claims 1 to 6, **characterised in that** the recording axes used are independent of one another, preferably orthogonal to one another.

8. Method according to one of claims 1 to 7, **characterised in that** the diagnostic imaging installation (I) is an x-ray computed tomograph (1) or a magnetic resonance tomograph or a positron emission tomograph.

9. Method according to one of claims 1 to 8, **characterised in that** the radiotherapy installation (II) is a radiotherapy installation (11) based on ionising radiation.

10. Method according to one of claims 1 to 9, **characterised in that** reference recordings (A) including the camera settings of the diagnostic imaging installation (I) are transmitted via a remote data link to the radiotherapy installation (II), wherein the radiotherapy installation (II) adjusts its camera (3.1, 3.2) automatically according to the transferred camera settings of the diagnostic imaging installation (I).

11. Method according to one of claims 1 to 10, **characterised in that** the reference recordings (A) of the diagnostic imaging installation (I) are brought into spatial correlation with diagnostic tomography recordings (CT1, CT2, CT3) of this installation, and in the radiotherapy installation (II) the spatial correlation of the diagnostic tomography recordings (CT1, CT2, CT3) to the current recording (B) is again produced in order to achieve a more accurate positioning of a focus of a disease to be treated.

12. Diagnostic imaging installation (I) and radiotherapy installation (II) with:
- a positioning apparatus (13) for the relative positioning of the patient and installation (I, II),
- at least one camera (3.1, 3.2), sensitive in the visible and/or infrared wavelength range, for checking the position of a patient to be treated and for creating at least two recordings (A, B) in at least two independent recording axes,
- a storage means for storing the recordings (A) and
- a monitor (8) for displaying these recordings, wherein
- means for comparing (14) currently created recordings (B) with reference recordings (A) created and stored in a previous session and
- means for displaying a difference (8) between reference recordings (A) and current recordings (B), wherein the recording of the reference recordings (A) and the recording of the current recordings (B) take place respectively in the diagnostic imaging installation (I) and in the radiotherapy installation (II), and
- the means (14) for comparing the current recordings (B) with the reference recordings (A) has a computer system (15) with a computer program for carrying out the method according to one of claims 1 to 11 and for displaying image differences (C) on the monitor (8), wherein the at least one camera for generating the reference recordings (A) and current recordings (B) is movably attached, preferably controlled automatically, in the space and/or in its orientation.

13. Diagnostic imaging installation (I) and radiotherapy installation (II) according to claim 12, **characterised in that** transmit and/or receive apparatuses (17, 18) for a data network are provided for transmitted images (A) and if necessary their camera settings.

14. Diagnostic imaging installation (I) and radiotherapy installation (II) according to one of claims 12 to 13, **characterised in that** at least two cameras (3.1, 3.2) are attached for generating the reference recordings (A) and/or current recordings (B), wherein their recording axes are arranged independently of one another, preferably orthogonally to one another.

## Revendications

1. Procédé de même repositionnement relatif répété d'un patient ( P ) par rapport à une installation ( 1 ) de diagnostic donnant une image et à une installation ( II ) de radiothérapie rapporté au système ( K₁, K₂ ) de coordonnées de l'installation ( I, II ) respective, dans lequel
- on réalise, dans au moins deux positions différentes d'un appareil photographique et dans des axes de prise de vue différents, au moins deux prises de vue ( A ) de référence du patient ( P ) ou d'une partie du patient ( P ) dans une première séance dans l'installation ( I ) de diagnostic donnant une image avec un réglage de l'appareil photographique par rapport au système ( K₁ ) de coordonnées de l'installation ( I ) de diagnostic donnant une image et on les mémorise,
- dans une autre séance, on réalise, dans l'installation ( II ) de radiothérapie, des prises de vue ( B ) instantanées dans la même position de l'appareil photographique par rapport au système ( K₂ ) de coordonnées de l'installation ( II ) de radiothérapie, dans lequel
- on superpose au moins deux prises de vue ( B ) instantanées à des prises de vue ( A ) de référence antérieures dans le même réglage de l'appareil photographique, de manière à pouvoir détecter des différences entre les prises de vue ( C ),
- on modifie ensuite la position ( P_{x,y,z} ) du patient ( P ) par rapport à l'installation ( II ) de radiothérapie jusqu'à minimiser les différences ( C ) entre les prises de vue ( A ) de référence et les prises de vue ( B ) instantanées pour la nouvelle position du patient et
- avant de superposer les prises de vue ( A ) de référence et les prises de vue ( B ) instantanées, on extrait les contours des prises de vue ( A, B ) et
- pour au moins une prise de vue ( A ) de référence, on mémorise la position et/ou la distance focale de l'appareil photographique ( 3.1, 3.2 ) et/ou l'axe de prise de vue par rapport à l'installation ( I ) de diagnostic donnant une image et, avant de produire des prises de vue ( B ) instantanées, on dispose le au moins un appareil photographique ( 3.1, 3.2 ) conformément aux données mémorisées.

2. Procédé suivant la revendication 1, **caractérisé en ce qu'**on mémorise numériquement les prises de vue ( A, B ) et on représente au moins les images superposées des prises de vue ( C ) sur un moniteur ( 8 ) de contrôle.

3. Procédé suivant la revendication 1 ou la revendication 2, **caractérisé en ce qu'**on produit la superposition ( C ) des prises de vue ( A ) de référence et des prises de vue ( B ) instantanées par un procédé de soustraction.

4. Procédé suivant l'une des revendications 1 à 3, **caractérisé en ce qu'**on produit la superposition ( C ) des prises de vue ( A ) de référence et des prises de vue ( B ) instantanées par un procédé d'addition.

5. Procédé suivant l'une des revendications 1 à 4, **caractérisé en ce qu'**on produit la superposition ( C ) des prises de vue ( A ) de référence et des prises de vue ( B ) instantanées par un procédé mixte.

6. Procédé suivant l'une des revendications 1 à 5, **caractérisé en ce que**, avant de superposer les prises de vus ( A ) de référence et les prises de vue ( B ) instantanées, a lieu un traitement ( 14 ) d'image pour avoir une représentation meilleure des différences d'image.

7. Procédé suivant l'une des revendications 1 à 6, **caractérisé en ce que** les axes de prises de vue utilisées sont indépendants l'un de l'autre, en étant de préférence orthogonaux l'un à l'autre.

8. Procédé suivant l'une des revendications 1 à 7, **caractérisé en ce que** l'installation ( 1 ) de diagnostic donnant une image est un tomographe ( 1 ) à ordinateur de rayons X ou un tomographe à résonance magnétique ou un tomographe à émission de positrons.

9. Procédé suivant l'une des revendications 1 à 8, **caractérisé en ce que** l'installation ( 2 ) de radiothérapie est une installation ( 2 ) de radiothérapie à base d'un rayonnement ionisant.

10. Procédé suivant l'une des revendications 1 à 9, **caractérisé en ce qu'**on transmet à l'installation ( 2 ) de radiothérapie par une liaison de données à distance des prises de vue ( A ) de référence, y compris les réglages d'appareils photographiques de l'installation ( I ) de diagnostic donnant une image, l'installation ( 2 ) de radiothérapie réglant son appareil photographique ( 3.1, 3.2 ) automatiquement, conformément aux réglages d'appareils photographiques transmis de l'installation ( 1 ) de diagnostic donnant une image.

11. Procédé suivant l'une des revendications 1 à 10, **caractérisé en ce qu'**on met en corrélation dans l'espace les prises de vue ( A ) de référence de l'installation ( I ) de diagnostic donnant une image avec des prises de vue ( CT1, CT2, CT3 ) panoramiques de diagnostic de cette installation et, dans l'installation ( 2 ) de radiothérapie, on produit à nouveau la corrélation dans l'espace des prises de vue ( CT1, CT2, CCT3 ) panoramiques de diagnostic avec la prise de vue ( V ) instantanée pour obtenir un positionnement plus précis d'un foyer de maladie à traiter.

12. Installation ( I ) de diagnostic donnant une image et installation ( II ) de radiothérapie, comprenant :
- un dispositif ( 13 ) de positionnement pour le positionnement relatif d'un patient et d'une installation ( I, II ),
- au moins un appareil photographique ( 3.1, 3.2 ) sensible dans le domaine de longueur d'onde visible et/ou infrarouge pour contrôler la position d'un patient à traiter et pour produire au moins deux prises de vue ( A, B ) dans au moins deux axes de prise de vue indépendants,
- un moyen de mémorisation pour mémoriser les prises de vue ( A ) et
- un moniteur ( 8 ) de représentation de ces prises de vue, dans laquelle
- il y a des moyens de comparaison ( 14 ) de prises de vue ( B ) produites instantanément à des prises de vue ( A ) de référence produites dans une séance antérieure et mémorisées et
- il y a des moyens de représentation d'une différence ( 8 ) entre des prises de vue ( A ) de référence et des prises de vue ( B ) instantanées, l'enregistrement des prises de vue ( A ) de référence ayant lieu dans l'installation ( I ) de diagnostic donnant une image et l'enregistrement des prises de vue ( B ) instantanées ayant lieu dans l'installation ( II ) de radiothérapie et
- le moyen ( 14 ) de comparaison des prises de vue ( B ) instantanées aux prises de vue ( A ) de référence comprend un système ( 15 ) d'ordinateur ayant un programme d'ordinateur pour effectuer le procédé suivant l'une des revendications 1 à 11 et pour représenter des différences ( C ) d'image sur le moniteur ( 8 ), le au moins un appareil photographique étant, pour la production des prises de vue ( A ) de référence et des prises de vue ( B ) instantanées, monté mobile dans l'espace et/ou dans son orientation, en étant de préférence commandé automatiquement.

13. Installation ( I ) de diagnostic donnant une image et installation ( II ) de radiothérapie suivant la revendication 12, **caractérisée en ce qu'**il est prévu des dispositifs ( 17, 18 ) d'émission et/ou de réception vers un réseau de données pour des images ( A ) transmises et, le cas échéant, leurs réglages d'appareil photographique.

14. Installation ( I ) de diagnostic donnant une image et installation ( II ) de radiothérapie suivant l'une des revendications 12 à 13, **caractérisée en ce qu'**il y a au moins deux appareils photographiques ( 3.1, 3.2 ) de production de prises de vue ( A ) de référence et/ou de prises de vue ( B ) instantanées, leurs axes de prise de vue étant disposés indépendamment l'un de l'autre, en étant de préférence orthogonaux entre eux.
